# EUROPEAN PATENT APPLICATION

(11) **EP 3 508 146 A1**
(43) Date of publication of application: **10.07.2019**
(21) Application number: 17845219.9
(22) Date of filing: 16.08.2017
(51) Int. Cl.: A61B 17/32, A61B 17/16

(54) **ULTRASONIC BONE SCALPEL HEAD**

(30) Priority: 31.08.2016 CN 201621016056 U
(71) Applicant: Jiangsu SMTP Technology Co., Ltd., Zhangjiagang, Jiangsu 215634 (CN)
(72) Inventor: LI, Weixin, Jiangsu 215634 (CN); ZHAN, Songtao, Jiangsu 215634 (CN); CAO, Qun, Jiangsu 215634 (CN)
(74) Representative: Daub, Thomas
(86) International application number: PCT/CN2017/097687
(87) International publication number: WO 2018/040917

(57) **Abstract**

Disclosed is a tool bit for an ultrasonic osteotome, comprising a head portion (1) of the tool bit, an arbor (2) and a tail portion (3). One end of the arbor (2) is connected with the head portion (1) of the tool bit, and the other end of the arbor (2) is connected with the tail portion (3). The tail portion (3) is connected with an ultrasonic main machine through an ultrasonic transducer. The head portion (1) of the tool bit has a flat structure. A front end surface of the flat structure is a smoothly curved surface, and an upper surface or both an upper and a lower surface of the flat structure are formed with knurls, which extends from a front end of the flat structure towards the arbor and covers the flat structure to form a flat-file type tool bit. The tool bit of the invention can be inserted to a small bone gap for a single side or double side grinding, and has simple structure, is easy for manufacture, convenient for use and has high safety.

## Description

### TECHNICAL FIELD

The present disclosure relates to the technical field of medical instruments, and in particular to a tool bit for an ultrasonic osteotome.

### BACKGROUND

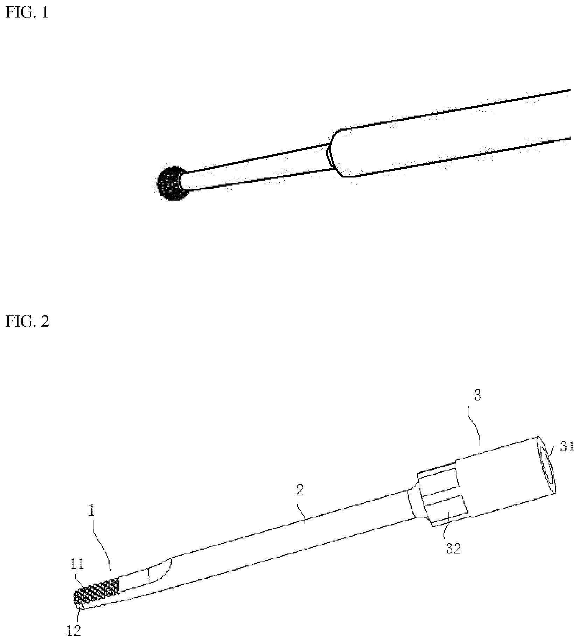

In modern society, with the development of medical technology, orthopedic surgery shows a trend of diversity. Accordingly, when a surgery is performed, it is necessary to use surgical tool bits of different shapes to perform cutting, grinding, scraping, clamping and other operations on an affected area according to different orthopedic conditions. During an orthopedic surgery, ultrasonic osteotomes are often used to perform cutting, grinding, planing, scraping or arbitrary shaping on bones. At present, a tool bit for an ultrasonic osteotome for grinding mainly has a spherical structure, as shown in Fig. 1. Although this type of tool bits for an ultrasonic osteotome can meet a part of grinding demands, on some occasions, such as for the grinding of a lumbar intervertebral disc endplate and the like, due to limited space, it is usually difficult to accomplish a bone grinding work.

### BRIEF SUMMARY

In order to solve the above technical problems in the art, the present invention provides a tool bit for an ultrasonic osteotome, which can be inserted into a small bone gap for a single-side or double-side grinding to enlarge exposed area.

In order to solve the above technical problems, the tool bit for an ultrasonic osteotome according to embodiments of the present invention comprises a head portion of the tool bit, an arbor and a tail portion, with one end of the arbor is connected with the head portion of the tool bit, and the other end of the arbor is connected with the tail portion, wherein the head portion of the tool bit has a flat structure, a front end surface of the flat structure is a smoothly curved surface, and an upper surface or both the upper and lower surfaces of the flat structure are formed with knurls, which extends from a front end of the flat structure toward the arbor and covers the flat structure to form a flat-file type tool bit.

In the tool bit for an ultrasonic osteotome of the present disclosure, preferably, when both the upper and lower surfaces of the flat structure are formed with knurls, the upper surface and the lower surface of the flat structure are formed with knurls of different tooth shapes, or knurls of same tooth shape but of different depths and different tooth pitches.

In the tool bit for an ultrasonic osteotome of the present disclosure, preferably, the upper surface and the lower surface of the flat structure are parallel, or the upper surface and the lower surface are inclined with respect to each other and form an included angle therebetween, so that the flat structure is thin at a front end portion and becomes thicker gradually in a direction of the arbor.

In the tool bit for an ultrasonic osteotome of the present disclosure, preferably, the flat structure is an elliptical flat structure or a rectangular flat structure, and a side surface of the elliptical flat structure is a smoothly curved surface.

In the tool bit for an ultrasonic osteotome of the present disclosure, preferably, the upper surface of the flat structure of the head portion of the tool bit is transitioned to the arbor smoothly by a curved surface.

In the tool bit for an ultrasonic osteotome of the present disclosure, preferably, the head portion of the tool bit is connected to the arbor through a thin cylindrical connecting portion, and an outer diameter of the thin cylindrical connecting portion is smaller than an outer diameter of the arbor.

In the tool bit for an ultrasonic osteotome of the present disclosure, preferably, the upper and lower surfaces of the flat structure of the head portion of the tool bit are smoothly transitioned by a straight plane and the thin cylindrical connecting portion, respectively, the thin cylindrical connecting portion and the arbor are smoothly connected by a curved transition surface.

In the tool bit for an ultrasonic osteotome of the present disclosure, preferably, the arbor and the tail portion are smoothly connected by a curved transition surface, and the other end of the tail portion is provided with a connection thread to be connected with an ultrasonic transducer.

In the tool bit for an ultrasonic osteotome of the present disclosure, preferably, the connection thread is an internal connection thread, and an outer surface of the tail portion is further provided with operating positions for a hex wrench.

In the tool bit for an ultrasonic osteotome of the present disclosure, preferably, the tool bit for an ultrasonic osteotome is of an integral structure or a split assembly structure.

In the tool bit for an ultrasonic osteotome of the present disclosure, preferably, the tail portion is connected with an ultrasonic main machine through an ultrasonic transducer.

In the tool bit for an ultrasonic osteotome of the present disclosure, preferably, a rear connection end of the head portion of the tool bit, the arbor and the tail portion all have hollow structure and communicate with each other, and the head portion of the tool bit has one aperture positioned at the rear of the upper surface or the lower surface of the flat structure of the head portion of the tool bit, or the head portion of the tool bit has two apertures positioned at the rear of the upper surface and the lower surface of the flat structure of the head portion of the tool bit.

The tool bit for an ultrasonic osteotome of the embodiments of the invention may have the following beneficial effects:
(1) The tool bit for an ultrasonic osteotome has a simple structure and is easy for machining.
(2) A tip end of the head portion of the tool bit has a smoothly curved surface, which can avoid scratching of soft tissues by the tip end, thereby improving safety.
(3) A flat-file type tool bit with various forms of file teeth is provided. When there are teeth on two sides, the tooth profile, the tooth depth or the tooth size of the two sides may be different, which is suitable and convenient for use in different occasions.
(4) The tool bit can be inserted into a small bone gap for a single-side or double-side grinding, and the connection way between the head portion of the tool bit and the arbor enables a wide visual field during surgery.
(5) The tool bit for an ultrasonic osteotome has a hollow structure, and the aperture on the head portion of the tool bit is located at the rear of the flat structure of the head portion of the tool bit, which can ensure that perfusate flows out from the rear surface of the head portion of the tool bit. During the operation, the entire head portion of the tool bit and the contacted tissue are completely exposed to the perfusate for cleaning and cooling in real time, ensuring that the excised bone fragments are immediately discharged, the visual field at the incision is clear and clean, and the remaining tissue to be retained is protected from being hurt, especially during a grinding operation. This further reduces the risk of surgery and improves the safety and success rate of an operation.

### BRIEF DESCRIPTION OF THE DRAWINGS

Fig. 1 is a schematic structural view of a spherical tool bit in the prior art;
Fig. 2 is a schematic perspective view of a tool bit for an ultrasonic osteotome according to a first embodiment of the present invention;
Fig. 3 is a front elevational view of the tool bit for an ultrasonic osteotome according to the first embodiment of the present invention;
Fig. 4 is a bottom plan view of the tool bit for an ultrasonic osteotome according to the first embodiment of the present invention;
Fig. 5 is a schematic perspective view of the tool bit for an ultrasonic osteotome according to a second embodiment of the present invention;
Fig. 6 is a front elevational view of the tool bit for an ultrasonic osteotome according to the second embodiment of the present invention;
Fig. 7 is a top plan view of the tool bit for an ultrasonic osteotome according to the second embodiment of the present invention;
Fig. 8 is a schematic perspective view of the tool bit for an ultrasonic osteotome according to a third embodiment of the present invention;
Fig. 9 is a front elevational view of the tool bit for an ultrasonic osteotome according to the third embodiment of the present invention;
Fig. 10 is a bottom plan view of the tool bit for an ultrasonic osteotome according to the third embodiment of the present invention;
Fig 11 is a rear elevational view of the tool bit for an ultrasonic osteotome according to the third embodiment of the present invention;
Fig. 12 is a schematic perspective view of a tool bit for an ultrasonic osteotome according to a fourth embodiment of the present invention;
Fig. 13 is a front elevational view of the tool bit for an ultrasonic osteotome according to the fourth embodiment of the present invention;
Fig. 14 is a bottom plan view of the tool bit for an ultrasonic osteotome according to the fourth embodiment of the present invention; and
Fig. 15 is a rear elevational view of the tool bit for an ultrasonic osteotome according to the fourth embodiment of the present invention.

### Reference numerals:

1∼head portion of the tool bit; 11∼knurls; 12∼curved surface; 2∼arbor; 3∼tail portion; 31∼connection thread; 32∼wrench position; 4∼thin cylindrical connecting portion; 5∼aperture.

### DETAILED DESCRIPTION OF THE INVENTION

Exemplary embodiments of the present invention will be described hereinafter clearly and completely with reference to the attached drawings. Apparently, the embodiments described herein are only portions of embodiments of the invention, rather than all embodiments of the invention. It is intended that all other embodiments obtained by those skilled in the art according to the disclosed embodiments without inventive labor are all within the scope of the present invention.

In the description of the present invention, it is to be noted that the terms of "center", "upper", "lower", "left", "right", "vertical", "horizontal", "internal", "external" and the like simply indicate orientational or positional relationship based on the accompanying drawings and are used only for the purpose of facilitating and simplifying the description of the invention, rather than specifying or implying that any devices or elements indicated must have a certain orientation, be configured or operate in a certain orientation. Therefore, these terms will not be interpreted as limiting the present invention. Further, the terms of "first", "second" and "third" and the like are only used for describing purpose, rather than being interpreted as specifying or implying relative importance.

In the description of the present disclosure, it is to be noted that, unless otherwise specified or defined clearly, the term of "attach", "connect to", "connect with", "couple" and the like should be interpreted broadly. For example, they may refer to fixed connection, or detachable connection, or integral connection; they may refer to mechanical connection, or electrical connection; they may refer to direct connection, or indirect connection through an intermediate agent, or internal communication between two components. For those skilled in the art, the specific meaning of these terms in the present disclosure may be understood in combination with specific situations or contexts.

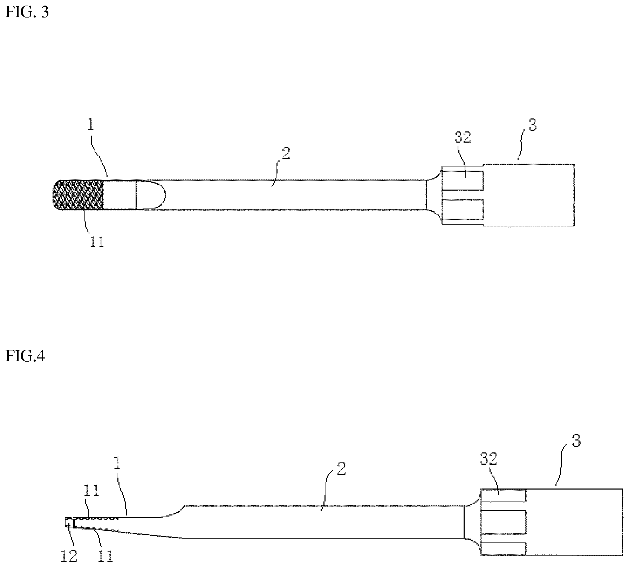

The invention will be further described in detail below with reference to specific embodiments and drawings. Fig. 2 is a schematic perspective view of a tool bit for an ultrasonic osteotome according to a first embodiment of the present invention. Fig. 3 is a front elevational view of the tool bit for an ultrasonic osteotome according to the first embodiment of the present invention. Fig. 4 is a bottom plan view of the tool bit for an ultrasonic osteotome according to the first embodiment of the present invention. As shown in Figs. 2 to 4, the tool bit for an ultrasonic osteotome according to the first embodiment of the present invention comprises a head portion 1 of the tool bit, an arbor 2, and a tail portion 3. One end of the arbor 2 is connected with the head portion 1 of the tool bit, and the other end of the arbor 2 is connected with the tail portion 3. The tail portion 3 is connected to a specific ultrasonic main machine through an ultrasonic transducer.

As shown in Figs. 2 to 4, the head portion 1 of the tool bit portion has a flat structure. In the tool bit for an ultrasonic osteotome according to the first embodiment of the present invention, the head portion 1 of the tool bit 1 has a rectangular flat structure. A front end face of the flat structure, i.e, a tip end portion is a curved surface 12, and the curved surface 12 is machined into a smooth surface to prevent scratching of soft tissue. There are knurls 11 formed on an upper surface or both an upper and a lower surface of the flat structure, and the knurls 11 extends from a front end of the flat structure in a direction of the arbor to cover the flat structure so as to form a planer file type tool bit. The knurls, i.e., file teeth, may be of a single-toothed profile, double-toothed profile, or other commonly used tooth profile for a filing. When there are knurls formed on both sides of the flat-file type tool bit, the tooth profiles on the two sides may be different. Alternatively, the tooth profiles may be the same but the tooth depth and the tooth size on two sides are different. This makes it easy to be adapted to more occasions. The upper surface and the lower surface of the flat structure may be parallel or may have an included angle tapering to a certain degree. When the upper surface and the lower surface of the flat structure have an included angle of a certain tapering degree, it is revealed that the flat structure is thin at the front end portion, and then becomes thicker gradually along with extending toward the direction of the arbor, thereby forming an cutting edge structure which is thin in front portion and thick in rear portion. This makes it possible to insert into a small bone gap for grinding operation. The arbor 2 and the tail portion 3 are connected by transition of a curved surface. The other end of the tail portion 3 is provided with a connection thread 31 for connecting with an ultrasonic transducer. In the embodiment, the connection thread 31 is designed as an internal connection thread, and the outer surface of the tail portion 3 is further provided with operating positions 32 for a hex wrench to facilitate a fastening operation. The head portion 1 of the tool bit and the arbor 2 may be of an integral structure or a split assembly structure. In the present embodiment, the head portion 1 of the tool bit and the arbor 2 are of an integral structure, and the upper surface of the flat structure and the arbor 2 are smoothly transitioned through a curved surface. The arbor 2 and the tail portion 3 are of an integral structure or a split assembly structure. In the present embodiment, the arbor 2 and the arbor 3 are of an integral structure machined from a cylindrical stepped rod.

In operation, the tool bit for an ultrasonic osteotome of the present invention is connected to a specific ultrasonic transducer through the connection thread 31 on the tail portion 3 thereof, and is tightened with a corresponding wrench, and then the ultrasonic transducer is connected to a specific ultrasonic main machine to be ready for work.

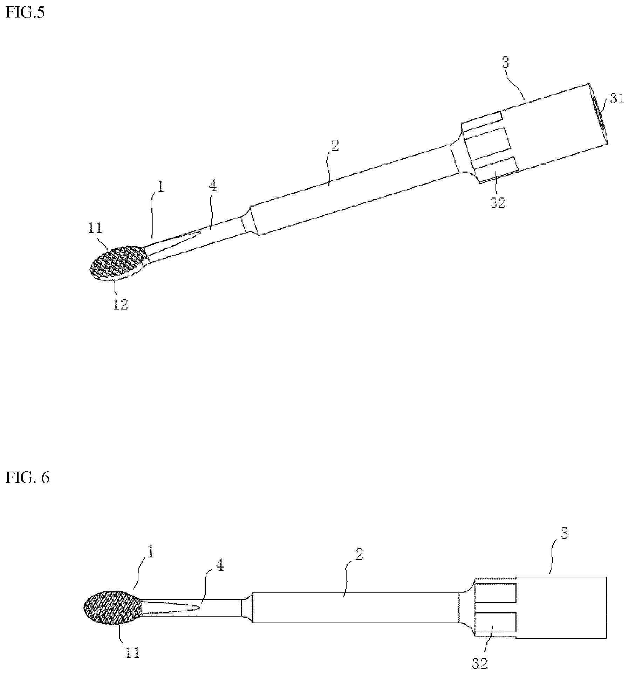

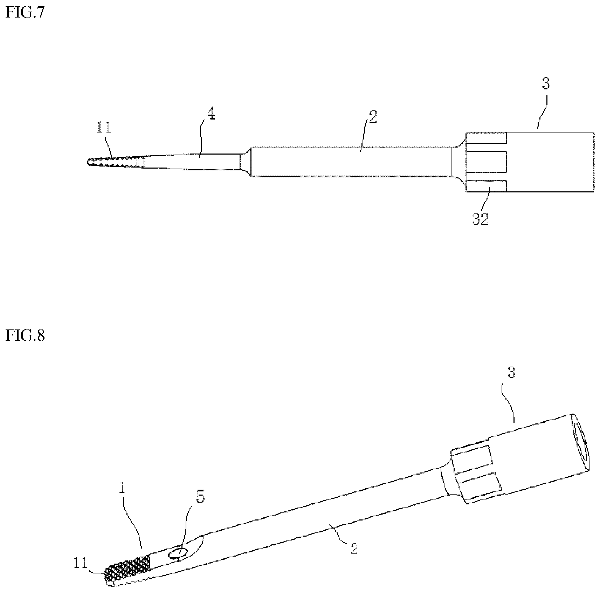

Figs. 5 to 7 show a second embodiment of a tool bit for an ultrasonic osteotome of the present invention. Fig. 5 is a schematic perspective view of the tool bit for an ultrasonic osteotome according to the second embodiment of the present invention. Fig. 6 is a front elevational view of the tool bit for an ultrasonic osteotome according to the second embodiment of the present invention. Fig. 7 is a top plan view of the tool bit for an ultrasonic osteotome according to the second embodiment of the present invention. As shown in Figs. 5 to 7, in the tool bit for an ultrasonic osteotome according to the second embodiment of the present invention, the head portion 1 of the tool bit has an elliptical flat structure. The elliptical flat structure has a side surface machined into a smoothly curved surface 12 to prevent scratching of soft tissue by edges of the side surface. The head portion 1 of the tool bit is connected to the arbor 2 via a thin cylindrical connecting portion 4. An outer diameter of the thin cylindrical connecting portion 4 is smaller than an outer diameter of the arbor 2. Such a shape is more advantageous for the visual field at the time of surgery. The upper and lower surfaces of the elliptical flat structure each are smoothly transitioned by a straight plane and the thin cylindrical connecting portion 4, so as to facilitate a flexible use of the head portion of the tool bit without obstruction. The thin cylindrical connecting portion 4 and the arbor 2 are connected by way of transition of a curved surface. The thin cylindrical connecting portion 4 and the arbor 2 may be of an integral structure or of a split assemble structure. In the present embodiment, the thin cylindrical connecting portion 4 and the arbor 2 are of an integral structure, and are machined by a cylindrical stepped rod.

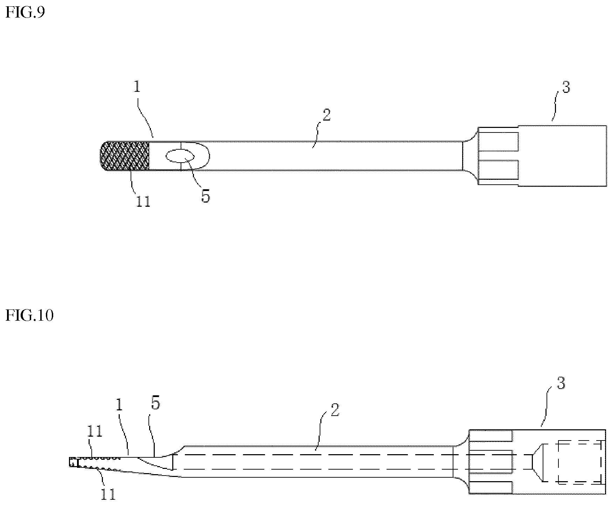

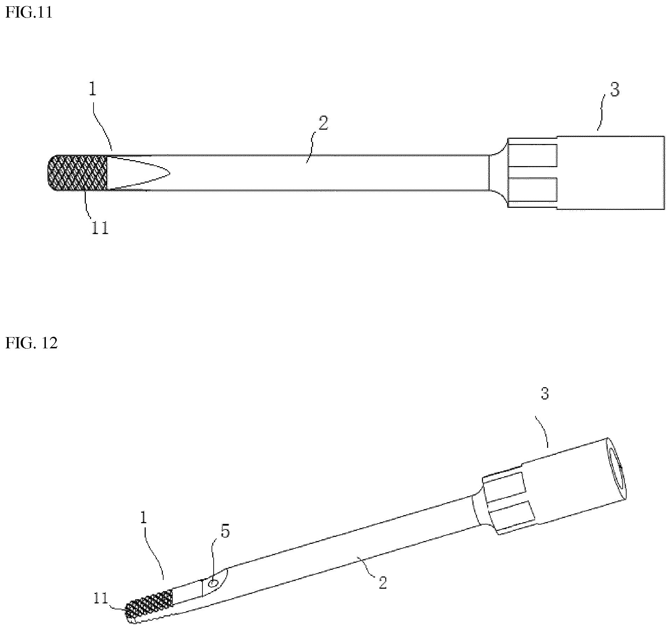

Figs.8 to 11 show a third embodiment of a tool bit for an ultrasonic osteotome of the present invention. Fig. 8 is a schematic perspective view of the tool bit for an ultrasonic osteotome according to a third embodiment of the present invention. Fig. 9 is a front elevational view of the tool bit for an ultrasonic osteotome according to the third embodiment of the present invention. Fig. 10 is a bottom plan view of the tool bit for an ultrasonic osteotome according to the third embodiment of the present invention. Fig 11 is a rear elevational view of the tool bit for an ultrasonic osteotome according to the third embodiment of the present invention. As shown in Figs. 8 to 11, the tool bit for an ultrasonic osteotome according to the third embodiment of the present invention is substantially same as the tool bit for an ultrasonic osteotome according to the first embodiment of the present invention, except that a rear connection end of the flat structure of the head portion 1 of the tool bit, the arbor 2 and the tail portion 3 all have hollow structure and communicate with each other, and an aperture 5 on the head portion 1 of the tool bit is located at the rear of the upper surface of the flat structure to ensure timely discharge of excised bone fractures. When a perfusate is used, it is ensured that the perfusate flows out from a rear surface of the head portion 1 of the tool bit, so that the entire head portion 1 of the tool bit and the contacted tissue are completely exposed to the perfusate for cleaning and cooling in real time during operation, which ensures that the bone fragments cut off are discharged in time, the visual field at the incision is clear and clean, and the remaining tissue to be retained is protected from damage. These effects are more evident in a grinding operation, which further reduces the risk of surgery and improves the safety and success rate of the operation.

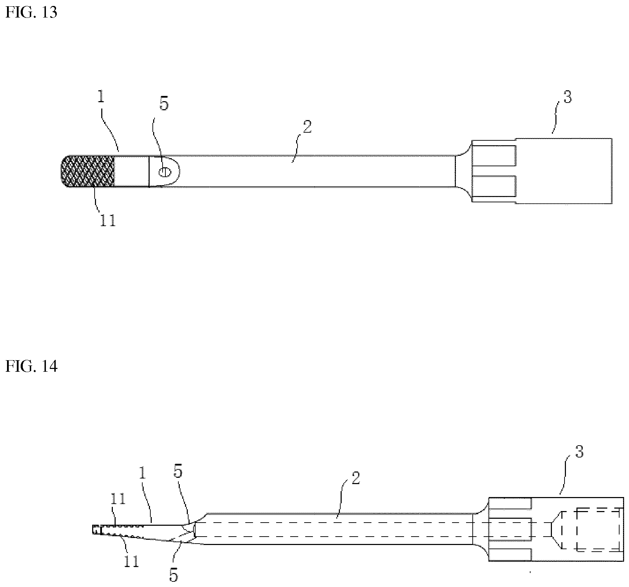

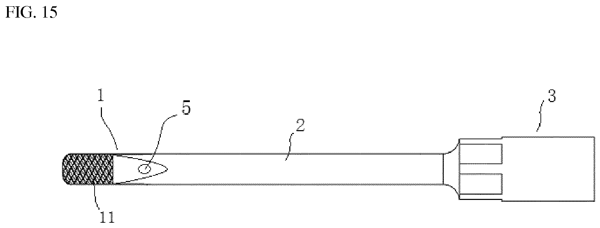

Figs. 12 to 15 show a fourth embodiment of a tool bit for an ultrasonic osteotome of the present invention. Fig. 12 is a schematic perspective view of a tool bit for an ultrasonic osteotome according to a fourth embodiment of the present invention. Fig. 13 is a front elevational view of the tool bit for an ultrasonic osteotome according to the fourth embodiment of the present invention. Fig. 14 is a bottom plan view of the tool bit for an ultrasonic osteotome according to the fourth embodiment of the present invention. Fig. 15 is a rear elevational view of the tool bit for an ultrasonic osteotome according to the fourth embodiment of the present invention. As shown in Figs. 12 to 15, the tool bit for an ultrasonic osteotome according to the fourth embodiment of the present invention is substantially same as the tool bit for an ultrasonic osteotome of the first embodiment of the present invention, except that a rear connection end of the flat structure of the head portion 1 of the tool bit, the arbor 2 and the tail portion 3 all have hollow structure and communicate with each other, and there are two apertures 5 on the head portion 1 of the tool bit. The two apertures 5 are respectively located at the rear of the upper and lower surfaces of the flat structure of the head portion 1 of the tool bit. Since there are two water apertures 5, i.e., the upper aperture and the lower aperture, the visual field at the incision can be made clear and clean in any cutting operation, and it is more advantageous to protect the remaining tissue from being damaged. In addition, when one water aperture is blocked or obstructed during operation, another aperture can still ensure a sufficient outflow of liquid. Further, with the liquid flowing out from two apertures 5, it can make liquid flow to the tissue more sufficiently. Therefore, the design of such a double water apertures structure makes the risk of surgery further reduced, and the safety and success rate of the operation are further improved.

The tool bit for an ultrasonic osteotome of the invention can concentrate all the energy generated by the ultrasonic transducer on the head portion of the tool bit (the most effective working part), so that the head portion of the tool bit as a front end portion of the tool bit has the strongest energy output and can achieve the strongest working effect. In addition, the tool bit for an osteotome of the invention is easy for manufacture. In addition, the tip end of the head portion of the tool bit of the present invention has a smoothly curved surface to avoid scratching of soft tissue by the tip end, thereby improving safety. The invention provides a flat-file type tool bit, which can adopt various forms of teeth. When there are file teeth formed at two sides, the tooth depth and the tooth size at the two sides may be different, which is suitable and convenient for use in different occasions.

The above embodiments are only used to describe the solutions of the present invention, rather than limiting the present invention. Although detailed descriptions of the invention are made with reference to the above embodiments, it would be appreciated by those skilled in the art that various changes or modifications to the above embodiments may be made or equivalent substitutions to portion of or all features in those embodiments may be made. Such changes, modifications or substitutions will not make the spirit of the relevant solutions depart from the scope of the technical solutions of the embodiments of the present invention.

## Claims

1. A tool bit for an ultrasonic osteotome comprising a head portion of the tool bit, an arbor and a tail portion, with one end of the arbor being connected with the head portion of the tool bit, and the other end of the arbor being connected with the tail portion, wherein
the head portion of the tool bit has a flat structure, a front end surface of the flat structure is a smoothly curved surface, and an upper surface or both an upper and a lower surface of the flat structure are formed with knurls, which extends from a front end of the flat structure towards the arbor and covers the flat structure to form a flat-file type tool bit.

2. The tool bit for an ultrasonic osteotome of claim 1, wherein
when both the upper and lower surfaces of the flat structure are formed with knurls, the upper surface and the lower surface of the flat structure are formed with knurls of different tooth shapes, or knurls of same tooth shape but of different depths and different tooth pitches.

3. The tool bit for an ultrasonic osteotome of claim 1 or 2, wherein
the upper surface and the lower surface of the flat structure are parallel, or the upper surface and the lower surface are inclined with respect to each other and form an included angle therebetween, so that the flat structure is thin at a front end portion and becomes thicker gradually with extending in a direction of the arbor.

4. The tool bit for an ultrasonic osteotome of claim 3, wherein
the flat structure is an elliptical flat structure or a rectangular flat structure, and a side surface of the elliptical flat structure is a smoothly curved surface.

5. The tool bit for an ultrasonic osteotome of claim 1, 2 or 4, wherein
the upper surface of the flat structure of the head portion of the tool bit is transitioned to the arbor smoothly by a curved surface.

6. The tool bit for an ultrasonic osteotome of claims 1, 2 or 4, wherein
the head portion of the tool bit is connected to the arbor through a thin cylindrical connecting portion, and an outer diameter of the thin cylindrical connecting portion is smaller than an outer diameter of the arbor.

7. The tool bit for an ultrasonic osteotome of claim 6, wherein
the upper and lower surfaces of the flat structure of the head portion of the tool bit each are smoothly transitioned by a straight plane and the thin cylindrical connecting portion, respectively, and the thin cylindrical connecting portion and the arbor are smoothly connected by a curved transition surface.

8. The tool bit for an ultrasonic osteotome of claim 1, 2, 4 or 7, wherein
the arbor and the tail portion are smoothly connected by a curved transition surface, and the other end of the tail portion is provided with a connection thread to be connected with an ultrasonic transducer.

9. The tool bit for an ultrasonic osteotome of claim 8, wherein
the connection thread is an internal connection thread, and an outer surface of the tail portion is further provided with operating positions for a hex wrench.

10. The tool bit for an ultrasonic osteotome of claim 1, 2, 4, 7 or 9, wherein
a rear connection end of the head portion of the tool bit, the arbor and the tail portion all have hollow structure and communicate with each other, and the head portion of the tool bit has one aperture positioned at the rear of the upper surface or the lower surface of the flat structure of the head portion of the tool bit, or the head portion of the tool bit has two apertures positioned at the rear of the upper surface and the lower surface of the flat structure of the head portion of the tool bit respectively.
